# EUROPÄISCHE PATENTSCHRIFT

(11) **EP 1 112 052 B1**
(45) Veröffentlichungstag und Bekanntmachung des Hinweises auf die Patenterteilung: **03.12.2003**
(21) Anmeldenummer: 99968621.5
(22) Anmeldetag: 09.09.1999
(51) Int. Cl.: A61J 3/10

(54) **VERFAHREN UND VORRICHTUNG ZUM HERSTELLEN VON TABLETTEN**
METHOD AND DEVICE FOR PRODUCING TABLETS
PROCEDE ET DISPOSITIF DE FABRICATION DE COMPRIMES

(30) Priorität: 09.09.1998 DE 19841244
(43) Veröffentlichungstag der Anmeldung: 04.07.2001
(73) Patentinhaber: ABBOTT GmbH & Co. KG, 65205 Wiesbaden (DE)
(72) Erfinder: ROSENBERG, Jörg, D-67158 Ellerstadt (DE); JOTTER, Karl-Ludwig, D-67454 Hassloch (DE); MAIER, Werner, D-67105 Schifferstadt (DE); TRAPP, Burkhard, D-67105 Schifferstadt (DE); TSCHOCHNER, Klaus, D-68535 Edingen-Neckerhausen (DE)
(74) Vertreter: Kinzebach, Werner, Dr.
(86) Internationale Anmeldenummer: EP9906672
(87) Internationale Veröffentlichungsnummer: WO00013647

(56) Entgegenhaltungen:
- EP-A- 0 240 906
- EP-A- 0 358 105
- DE-A- 19 539 361

## Beschreibung

Die vorliegende Erfindung betrifft ein Verfahren und eine Vorrichtung zum Herstellen von Tabletten mittels Schmelzextrusion.

Aus dem europäischen Patent EP-B-0 240 906 ist ein Verfahren zum Herstellen von Tabletten mittels Schmelzextrusion bekannt, bei dem man eine extrudierbare pharmazeutische Mischung erwärmt und in Form eines kontinuierlichen Produktstranges extrudiert, den noch formbaren Produktstrang zu einem kontinuierlichen Tablettenband verpreßt, wobei die einzelnen Tabletten des Bandes durch Produktstege miteinander verbunden sind, das Tablettenband abkühlen läßt und die Tabletten schließlich vereinzelt und entgratet.

Im Gegensatz zu konventionellen Verfahren, die auf dem verpressen von Pulvern oder Granulaten beruhen, wird beim Schmelzextrusionsverfahren eine wirkstoffhaltige Schmelze eines thermoplastischen, wasserlöslichen oder wasserquellbaren Polymers verarbeitet.

Dazu werden die einzelnen Komponenten zunächst vermischt und dann in einem Extruder aufgeschmolzen. Auch das Vermischen der Komponenten kann im Extruder erfolgen. Als Extruder kommen beispielsweise Einschneckenmaschinen, kämmende Schneckenmaschinen, Mehrwellenwextruder, insbesondere zweischnecken-Extruder, in Frage, die gleichsinnig oder gegensinnig drehend ausgebildet und gegebenenfalls mit Knetscheiben ausgerüstet sein können. Geeignete Extruder finden sich beispielsweise in der ZSK-Baureihe der Fa. Werner u. Pfleiderer.

Der Extruder kann mehrere Einfüllstutzen aufweisen. Es kann gegebenenfalls eine getrennte Zugabe von festen und flüssigen Mischungsbestandteilen vorgesehen sein. Außerdem können Anschlußstutzen zur Inertbegasung und/oder Entgasung vorgesehen sein. Da die Mischung der Bestandteile im Extruder erfolgt, kann normalerweise auf ein Vormischen verzichtet werden. Die erwärmte pharmazeutische Mischung wird über ein oder mehrere Düsen, beispielsweise Schlitzdüsen im Extruderkopf in Form von Produktsträngen oder -bändern ausgepreßt. Die Produktstränge oder -bänder werden dann Formgebungsmitteln zugeführt. Es sind verschiedene Mittel zur Tablettenformung aus wirkstoffhaltigen Schmelzen bekannt. Beispielsweise kann die Schmelze durch ein Kalandrierverfahren mittels gegenläufig rotierender Formwalzen zu Tabletten verpreßt werden. Dabei sind in einer oder in beiden Formwalzen der gewünschten Tablettenform entsprechende Vertiefungen vorgesehen. Es ist jedoch auch möglich, zwischen glatten Kalanderwalzen ein Band durchlaufen zu lassen, das Vertiefungen oder Öffnungen in der gewünschten Tablettenform aufweist. Das durch Kalandrieren hergestellte Tablettenband enthält die geformten Einzeltabletten, die üblicherweise durch feine Preßnähte oder Produktstege miteinander verbunden sind. Diese Produktstege können bei der Formgebung sogar nützlich sein, da sie das Herauslösen der Tablette aus der Form begünstigen. Zur Durchführung des herkömmlichen Schmelzextrusionsverfahrens sei neben der EP-B-0 240 906 auch auf die EP-B-0 240 904, EP-B-0 337 256 und die EP-B-0 358 105 verwiesen.

Zur Herstellung von Einzeltabletten aus dem so geformten Tablettenband läßt man das Band zunächst abkühlen und gibt die erkalteten Tablettenbänder in einen großen Kesselbehälter, der in Rotation versetzt wird. Durch die dadurch auftretende mechanische Belastung, die über die zugegebene Tablettenmenge, die Größe des Kessels und dessen Rotationsgeschwindigkeit in gewisssen Grenzen gesteuert werden kann, vereinzeln die großen, plattenförmigen Teile des Tablettenbandes schrittweise zu immer kleineren Aggregaten, bis schließlich nur noch ein hoher Anteil von sogenannten "Zwillingen" verbleibt, der im weiterem Prozeß zu Einzeltabletten vereinzelt. Durch die Kollision der Tabletten während der Rotation im Kessel werden die Reste der Preßnähte abgerieben, so daß die Tabletten gleichzeitig zur Vereinzelung auch entgratet werden.

Das bekannte Verfahren zur Herstellung von Einzeltabletten durch Schmelzextrusion ist jedoch mit Nachteilen behaftet. Während das Schmelzextrusionsverfahren ein kontinuierliches Verfahren zur Herstellung von Tabletten bereitstellt, erfolgt der abschließende Vereinzelungs- und Entgratungsvorgang absatzweise in Kesselbehältern. Da die Prozeßzeit im Kesselbehälter, insbesondere zum Vereinzeln der Tabletten aus dem Tablettenband, relativ groß ist, wird die mögliche Produktivität einer kontinuierlichen Tablettenherstellung durch Schmelzextrusion nicht ausgenutzt. Außerdem hat sich gezeigt, daß dieses einfache Vereinzelungs- und Entgratungsverfahren im Kesselbehälter nicht in allen Fällen erfolgreich ist. Insbesondere bei Tabletten, die eine Bruchkerbe zur leichten Teilbarkeit besitzen, führt das bekannte Verfahren zu einem hohen Anteil von zerbrochenen Tabletten, die mechanisch aussortiert werden müssen und die Ausbeute erheblich herabsetzen. Teilbare Tabletten, die eine Bruchkerbe aufweisen, werden jedoch in den letzten Jahren immer häufiger eingesetzt, da sich mit ihnen eine Dosisanpassung z.B. Kinder-/Erwachsenen-Dosierung, mit einer einzigen Tablette erreichen läßt.

Aufgabe der vorliegenden Erfindung ist es daher ein Verfahren zum Herstellen von Tabletten mittels Schmelzextrusion bereitzustellen, das einerseits eine höhere Produktivität ermöglicht und das außerdem eine Herstellung von leicht zerbrechlichen, beispielsweise teilbaren Tabletten ohne unannehmbar hohe Verlustrate gewährleistet. Aufgabe der vorliegenden Erfindung ist auch die Bereitstellung einer Vorrichtung zur Durchführung des Verfahrens.

Gelöst wird diese Aufgabe durch das Verfahren gemäß vorliegendem Hauptanspruch.

Gegenstand der vorliegenden Erfindung ist demnach ein Verfahren zur Herstellung von Tabletten mittels Schmelzextrusion, bei dem man in an sich bekannter Weise eine extrudierbare, vorzugsweise pharmazeutische Mischung erwärmt und in Form eines kontinuierlichen Produktstranges extrudiert, den noch verformbaren Produktstrang zu einem kontinuierlichen Tablettenband verpreßt, wobei die einzelnen Tabletten des Bandes durch Produktstege miteinander verbunden sind, das Tablettenband abkühlen läßt und die Tabletten schließlich vereinzelt und entgratet, das dadurch gekennzeichnet ist, daß man zunächst die Tabletten in einem kontinuierlichen Prozeß mechanisch vereinzelt, dann die vereinzelten Tabletten weiter transportiert und sie anschließend entgratet.

Erfindungsgemäß wird demnach vorgeschlagen, den bei bekannten Verfahren in einem Rundkessel stattfindenden, kombinierten Vereinzelungs- und Entgratungsvorgang in zwei separaten Teilschritten durchzuführen, wobei der Vereinzelungsvorgang kontinuierlich erfolgt.

Mit dem erfindungsgemäßen Verfahren sind zahlreiche Vorteile verbunden. Durch eine kontinuierliche Vereinzelung des Tablettenbandes zu Einzeltabletten kann dieser Teilschritt mit der gleichen Geschwindigkeit wie die Formung der Tabletten durch Schmelzextrusion erfolgen. Der anschließende Entgratungsschritt kann wie beim erfindungsgemäßen Verfahren in einem Kesselbehälter stattfinden. Bevorzugt werden an sich bekannte Dragierkessel oder für das Filmcoating gebräuchliche Maschinen (etwa der Dria-Coater der Firma Driam) eingesetzt. Da im Kessel keine Vereinzelung mehr erfolgen muß, ist dort die für die Entgratung benötigte Zeit deutlich verkürzt. Insgesamt ergibt sich somit eine Einsparung an Prozeßzeit gegenüber dem bekannten Verfahren.

Die erfindungsgemäß vorgesehene Trennung von Vereinzelung und Entgratung erlaubt außerdem eine spezifische Anpassung der beiden Prozesse an die jeweiligen Erfordernisse. Da im abschließenden Entgratungsschritt im Kessel nur noch die an den Einzeltabletten befindlichen Reste der Preßnaht entfernt werden müssen, kann die Entgratung unter schonenderen Bedingungen erfolgen. Beim Verfahren des Standes der Technik ist der Energieeintrag in den Kessel wesentlich höher, da dort vor allem auch die Vereinzelung der größeren Tablettenplatten erfolgen muß. Das erfindungsgemäße Verfahren ist daher vor allem bei der Herstellung von leicht zerbrechlichen Tabletten, wie beispielsweise von Tabletten mit Bruchkerben besonders vorteilhaft einsetzbar.

Zur Vereinzelung des Tablettenbandes nach dem erfindungsgemäßen Verfahren muß die Temperatur der Tabletten so weit erniedrigt sein, daß bei der Einwirkung einer mechanischen Kraft kein Verbiegen oder Verformen der eventuell noch plastischen Tabletten erfolgt.

Gemäß einer vorteilhaften Ausführungsform des erfindungsgemäßen Verfahrens läßt man zum Vereinzeln der Tabletten eine Kraft mit zur Ebene des abgekühlten Tablettenbandes senkrechter Komponente auf das Band einwirken. Dies führt unmittelbar zu einem Abknicken und anschließendem Brechen des Tablettenbandes an den dünnen Produktstegen, die die Einzeltabletten miteinander verbinden. Das Verfahren funktioniert besonders dann sehr effektiv, wenn die Temperatur des Tablettenbandes bereits so niedrig ist, daß die dünnen Produktstege oder Preßnähte nicht mehr plastisch sind, sondern eine gewisse Sprödigkeit aufweisen. Es ist jedoch auch möglich, eine Kraft in der Ebene des Tablettenbandes wirken zu lassen, so daß die Tabletten an den Produktstegen auseinander gerissen werden. Dies Variante des erfindungsgemäßen Verfahrens kann bereits dann eingesetzt werden, wenn die Temperatur des Tablettenbandes noch so hoch ist, daß die Produktstege noch eine gewisse Plastizität besitzen. Besonders vorteilhaft ist jedoch ein Verfahren, bei dem so wohl eine Kraft senkrecht zum Tablettenband als auch eine Kraft in der Ebene des Tablettenbandes wirkt.

Die senkrechte Kraftkomponente wird man bevorzugt durch Auslenken des erstarrten Tablettenbandes aus seiner Transportebene erzeugen, während die parallele Kraftkomponente durch Ausüben einer Zugkraft auf das erstarrte Tablettenband erzeugt wird.

Gegenstand der vorliegenden Erfindung ist auch eine Vorrichtung zum Herstellen von Tabletten, insbesondere zur Durchführung des oben beschriebenen erfindungsgemäßen Verfahrens. Die erfindungsgemäße Vorrichtung umfaßt wenigstens einen Extruder, ein dem Extruder nachgeordnetes Mittel zum Formen eines Tablettenbandes, ein dem Formungsmittel nachgeordnetes erstes Transportmittel für das Tablettenband und Mittel zum Vereinzeln und Entgraten der Tabletten. Die erfindungsgemäße Vorrichtung ist dadurch gekennzeichnet, daß die Mittel zum Vereinzeln und Entgraten der Tabletten wenigstens ein dem ersten Transportmittel nachgeordnetes Vereinzelungsmittel und wenigstens ein dem Vereinzelungsmittel nachgeordnetes und von diesem räumlich getrenntes Entgratungsmittel umfassen. Als Formungsmittel kommen in erster Linie zwei gegeneinander preßbare Formwalzen in Frage, wie sie in dem eingangs erwähnten europäischen Patent EP-B-0 240 906 beschrieben sind. Bei dem ersten Transportmittel kann es sich beispielsweise um ein Förderband handeln, das in erster Linie zur Abkühlung der zu einem Tablettenband verpreßten pharmazeutischen Schmelze dient.

Das sich an das erste Transportmittel anschließende Vereinzelungsmittel ist besonders vorteilhaft als Walzenanordnung ausgebildet. Gemäß einer einfachen Ausführungsform umfaßt das Vereinzelungsmittel wenigstens eine drehbare Walze zur Auslenkung des Tablettenbandes auf einer Transportebene des ersten Transportmittels. Auf dem ersten Transportmittel verfestigt sich die zunächst noch plastische Schmelze, so daß das erstarrte Tablettenband das Transportmittel in einer durch dieses vorgegebenen Ebene verläßt. Unmittelbar im Anschluß an das Transportmittel kann eine drehbare Walze angeordnet sein, die das starre Band beispielsweise nach unten ablenkt und so die im erfindungsgemäßen Verfahren vorgesehene Kraft senkrecht zum Tablettenband ausübt. Bei dieser Auslenkung brechen die nunmehr spröden Verbindungsstege zwischen den Tabletten. Bei einer vorteilhaften Ausführungsform der erfindungsgemäßen Vorrichtung sind die Vereinzelungsmittel als zwei gegeneinanderpreßbare, gegenläufig rotierende Walzen ausgebildet. Eine der Walzen läuft oberhalb und die andere Walze unterhalb des Tablettenbandes. Die Walzen können antreibbar ausgebildet sein. Die Umdrehungsgeschwindigkeit kann so hoch gewählt werden, daß sie größer als die Transportgeschwindigkeit des Tabletttenbandes auf dem ersten Transportmittel ist, so daß die Walzen eine Zugkraft in der Ebene des Tablettenbandes erzeugen. Die Walzen können so angeordnet sein, daß die Tangentialebene des Durchlaßspaltes für das Tablettenband einen Winkel mit der Ebene des Tablettenbandes auf dem Transportmittel bildet, so daß wiederum eine Auslenkung des erstarrten Bandes bewirkt wird, was zu einem Bruch des Tablettenbandes an den Produktstegen führt. Die als Vereinzelungsmittel verwendeten Walzen bzw. Walzenkombinationen können leicht an unterschiedliche Anforderungen spezieller Tablettenformulierungen angepaßt werden. Beispielsweise sind walzen mit unterschiedlicher Oberflächenstruktur einsetzbar. Es können beispielsweise Glattwalzen, walzen mit Bürsten- oder Nocken, mit Stegen oder anderen Strukturen verwendet werden. Durch Veränderung der Anordnung der Walzen, des Walzendurchmessers und des Anpreßdrucks kann die zum Vereinzeln aufgewendete Kraft beeinflußt werden. Es sind auch unterschiedliche Werkstoffkombinationen, beispielsweise Schaumstoffe, Kunststoffe, Gummi oder Edelstahl denkbar. Durch die Rotationsgeschwindigkeit der Walzen läßt sich insbesondere die Zugkraft in der Ebene des Tablettenbandes beeinflussen.

Gemäß einer bevorzugten Ausführungsform der Erfindung weist das erste Transportmittel zusätzliche Mittel zum Kühlen des extrudierten und geformten Tablettenbandes auf. wenn das Transportmittel beispielsweise als umlaufendes Förderband ausgebildet ist, können unterhalb des Obertrums des Bandes ein oder mehrere Kühlplatten vorgesehen sein. Durch die Länge des Transportbandes kann die Abkühlung den speziellen Erfordernissen der jeweiligen Tablettenformulierung angepaßt werden. Bei längeren Transportbändern kann man die Kühlstrecke in einzelne Zonen unterteilen, die separat gekühlt werden, so daß ein schrittweiser, gut kontrollierbarer Abkühlvorgang durchführbar ist.

Die bei den ersten Transportmitteln vorgesehene Kühlung kann aber auch durch eine Luftkühlung erfolgen. Dabei ist sowohl eine Kühlung von oben möglich, wobei man beispielsweise Kühlluft über das geformte Tablettenband streichen läßt. Es ist aber auch möglich, das Transportband perforiert auszubilden und eine Luftkühlung von unten vorzusehen. Die Kühlung des Tablettenbandes kann aber auch beispielsweise durch Besprühen mit Kühlwasser erfolgen.

Bei einer besonders bevorzugten Ausführungsform der erfindungsgemäßen Vorrichtung ist zwischen den Vereinzelungsmitteln und den Entgratungsmitteln ein zweites Transportmittel vorgesehen, das eine Rüttel- oder Vibrationseinrichtung umfaßt. Diese kann beispielsweise als Vibrationssieb ausgebildet sein. Nach dem Vereinzeln fallen die Tabletten auf ein Vibrationssieb und werden dort zum Entgratungsmittel transportiert. Bereits auf dem Vibrations-sieb werden größere Reste der Preßnähte von den Tabletten abgetrennt, so daß die Prozeßzeit für das Entgraten weiter verringert wird. Auch nach dem Vereinzeln eventuell noch vorhandene "Zwillinge", das heißt zwei noch durch Produktstege verbundene Tabletten, können auf dem vibrierenden zweiten Transportmittel getrennt werden.

Die vorliegende Erfindung wird in folgendem unter Bezugnahme auf ein in den beigefügten Zeichnungen dargestelltes Ausführungsbeispiel ausführlicher erläutert.

In den Zeichnungen zeigt:
- Figur 1: eine schematische Darstellung einer erfindungsgemäßen Vorrichtung zum Herstellen von Einzeltabletten mittels Schmelzextrusion;
- Figur 2: eine Aufsicht auf ein Tablettenband unmittelbar nach der Formung mit den Formkalanderwalzen; und
- Figur 3: einen Schnitt durch das Tablettenband der Fig. 2;
- Figur 4: eine Aufsicht auf eine Variante der Walzenanordnung der Vereinzelungsmittel;
- Figur 5: eine Aufsicht auf eine weitere Ausführungsform der Brechwalze der Vereinzelungsmittel.

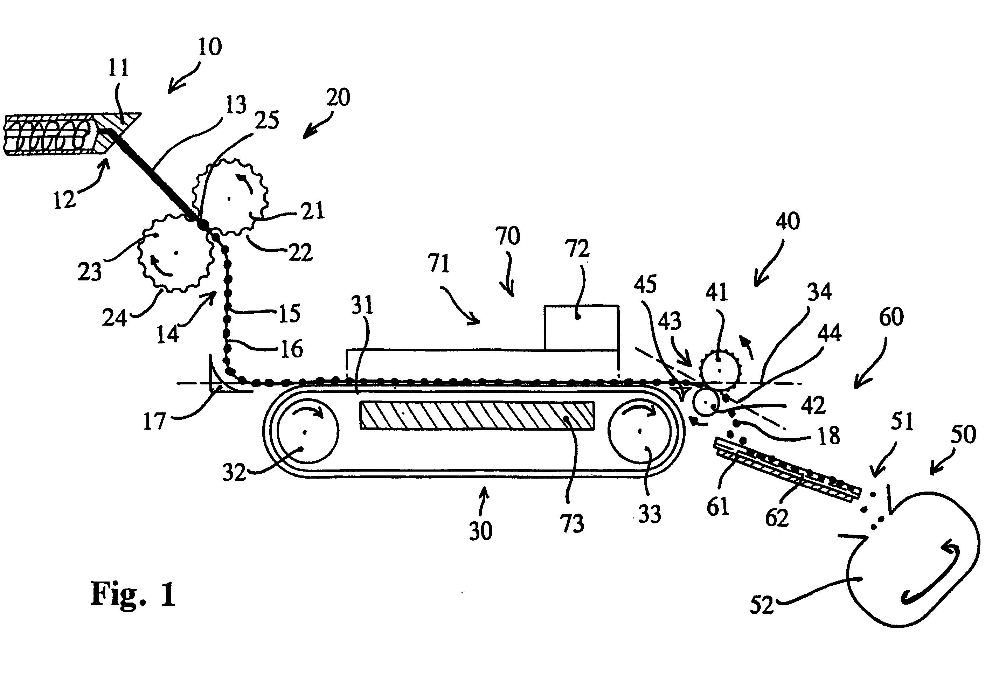

In Figur 1 ist eine bevorzugte Ausführungsform der erfindungsgemäßen Vorrichtung in der Übersicht dargestellt. Man erkennt den schematisch dargestellten Extruder 10, der zum Mischen und Aufschmelzen der pharmazeutischen Mischung dient. Ein Extruderkopf 11 des Extruders 10 weist eine Schlitzdüse 12 auf, aus der das bandförmige, plastische Extrudat ausgepreßt wird. Im noch plastischen Zustand gelangt das Extrudat 13, in das Formungsmittel 20, das im vorliegenden Fall aus zwei gegenläufig rotierenden Kalanderwalzen 21,23 gebildet wird. In den Oberflächen der Walzen sind Vertiefungen 22 bzw. 24 ausgespart, deren Form jeweils einer Hälfte der herzustellenden Tablette entspricht. Die Walzen werden so zueinander justiert, daß im Kontaktbereich jeweils zwei Formhälften exakt übereinstimmen. In diesem Kontaktbereich bilden die Walzen einen Einzugsspalt 25, in welchen Extrudat 13 gelangt und zu einem Tablettenband 14 verpreßt wird.

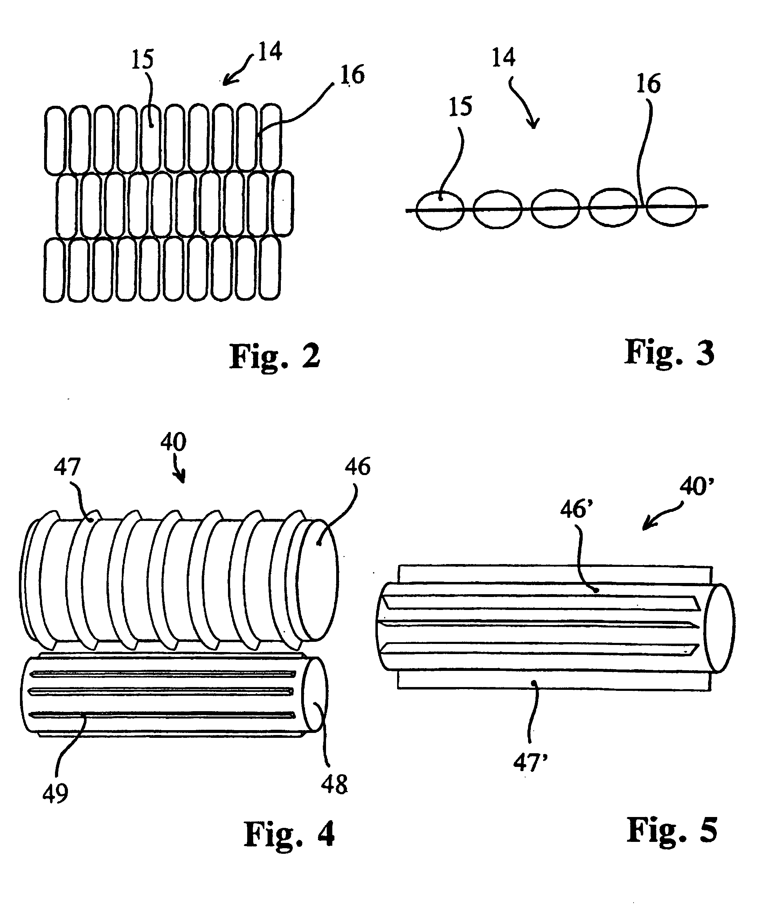

Das so hergestellte Tablettenband ist in den Figuren 2 und 3 detaillierter dargestellt. Die Tabletten 15 des Tablettenbandes 14 sind über Produktstege 16 miteinander verbunden. Wie insbesondere aus der Schnittdarstellung der Figur 3 hervorgeht, sind die Produktstege 16 gegenüber den Tabletten 15 sehr dünn.

Das Tablettenband gelangt gegebenenfalls über geeignete Umlenkeinrichtungen 17 auf ein erstes Transportmittel 30, das im vorliegenden Fall als Bandfördereinrichtung ausgebildet ist. Die Fördereinrichtung weist ein umlaufendes Förderband 31 und zwei Umlenkrollen 32, 33 auf. Im dargestellten Beispiel sind außerdem Kühlmittel 70 vorgesehen, wobei exemplarisch eine Umluftkühlung 71 mit einem Kühlaggregat 73 oberhalb des Tablettenbandes 14 und Kühlplatten 73 unterhalb des Tablettenbandes 14 dargestellt sind.

Die Länge des Förderbandes 31 ist - abhängig von den verwendeten zusätzlichen Kühlmitteln - so gewählt, daß die Produktstege 16 am Ende des Förderbandes so weit abgekühlt sind, daß sie bereits eine gewisse Sprödigkeit besitzen. Das weitgehend erstarrte Tablettenband auf dem Transportmittel 30 definiert eine - in Figur 1 gestrichelt dargestellte - Transportebene 34.

Unmittelbar an das erste Transportmittel 30 schließt sich das Vereinzelungsmittel 40 an, das im vorliegenden Fall aus zwei gegeneinander gepreßten Walzen 41, 42 besteht. Die obere Walze 41 ist als Noppenwalze ausgebildet, während die untere Walze als Glattwalze ausgebildet ist. Die Walzen sind in Transportrichtung des Bandes leicht gegeneinander versetzt und sind außerdem so angeordnet, daß der durch die Walzen definierte Spalt 43 unterhalb der Transportebene 34 des erstarrten Bandes liegt. Man erkennt, daß die - gestrichelt dargestellt - Tangentialebene 44 des Spaltes 43 einen Winkel mit der Transportebene 34 bildet, so daß ersichtlich wird, daß das erstarrte Band bei der Durchführung durch den Spalt 43 nach unten abgelenkt wird. Durch diese Ablenkung wird eine Kraft im wesentlichen senkrecht zur Ebene des Tablettenbandes 14 ausgeübt, was den Bruch der dünnen Produktstege 16 bewirkt. Zwischen den beiden Walzen 41 und 42 und dem Ende des Förderbandes 31 ist im dargestellten Beispiel noch eine Leiteinrichtung 45 für das Tablettenband vorgesehen.

Nach Durchlaufen der Vereinzelungsmittel liegen die Tabletten des Bandes als Einzeltabletten 18 vor, die teilweise noch mit Resten des Produktsteges umgeben sind. Die Einzeltabletten 18 fallen auf ein zweites Transportmittel 60, das hier als Rüttelsieb 61 ausgebildet ist. Das Rüttelsieb 61 führt die Einzeltabletten 18 in das Entgratungsmittel 50, das aus einer rotierenden Trommel 52 mit Einlaßöffnung 51 besteht. Beim Transport auf dem Rüttelsieb 61 brechen die Reste der Produktstege von den Einzeltabletten 18 ab und gelangen in eine Auffangrinne 62. Die Tabletten sind daher nur noch mit einem sehr dünnen Grat versehen, der nach kurzer Behandlungszeit in der Trommel 52 vollständig abgeschliffen ist.

In Figur 4 erkennt man eine weitere Variante der erfindungsgemäßen Walzenkombination der Vereinzelungsmittel 40. Die oberhalb des Tablettenbandes laufende Walze 46 weist Längsstege 47 auf, während die untere Walze 48 querverlaufende Stege 49 besitzt, die im wesentlichen senkrecht zu den Längsstegen 47 angeordnet sind.

Figur 5 schließlich, zeigt eine weitere Variante einer Brechwalze der Vereinzelungsmittel 40'. Die Walze 46' weist mehrere biegsame Kunststofflamellen 47' auf, die längs zur Achse der Walze 46' orientiert sind. Die Lamellen können beispielsweise auf den zylindrischen Walzengrundkörper aufgeklebt, auf geformt oder in dort ausgesparten Schlitzen festgeklemmt sein. Die Lamellenwalze 46' kann als einzelne Brechwalze oder zusammen mit einer ähnlich aufgebauten Gegenwalze oder eine Glattwalze eingesetzt werden.

Der Begriff "Tablette" im Sinne der vorliegenden Erfindung ist breitest möglich zu verstehen. Er ist weder an eine bestimmte Form noch eine bestimmte Anwendung gebunden. Er umfaßt daher beispielsweise Tabletten zur peroralen Anwendung aber auch Tabletten beispielsweise zur rektalen Anwendung in Form von Zäpfchen. Unter Tabletten sind hier auch alle Dosierungsformen zu verstehen, die zur Verwendung als Arzneimittel, Pflanzenbehandlungsmittel, Futtermittel und Nahrungsmittel, sowie zur Abgabe von Riechstoffen und Parfümölen geeignet sind.

Unter pharmazeutischen Wirkstoffen im Sinne der Erfindung sind alle Stoffe mit einer pharmazeutischen Wirkung und möglichst geringen Nebenwirkungen zu verstehen, so fern sie sich unter den Verarbeitungsbedingungen nicht zersetzen. Die Wirkstoffmenge pro Dosiseinheit und die Konzentration können je nach Wirksamkeit und Freisetzungsgeschwindigkeit in weiten Grenzen variieren. Einzige Bedingung ist dabei, daß sie zur Erzielung der gewünschten Wirkung ausreichen. So kann die Wirkstoffkonzentration im Bereich von 0,1 bis 95, vorzugsweise von 20 bis 80, insbesondere 30 bis 70 Gewichtsprozent liegen. Der Begriff Wirkstoff umfaßt im vorliegenden Zusammenhang auch beliebige Wirkstoffkombinationen. Wirkstoffe im Sinne der Erfindung sind beispielsweise auch Vitamine. Besonders bevorzugte Wirkstoffe sind Ibuprofen (als Racemat, Enantiomer oder angereichertes Enantiomer), Ketoprofen, Flurbiprofen, Acetylsalicylsäure, Verapamil, Paracetamol, Nifedipin und Captopril.

Das polymere Bindemittel muß in der Gesamtmischung aller Komponenten im Bereich von 50 bis 180 °C, vorzugsweise 60 bis 130 °C erweichen oder schmelzen. Die Glasübergangstemperatur der Mischung muß daher unter 180 °C, vorzugsweise unter 130 °C liegen. Erforderlichenfalls wird sie durch übliche, pharmakologisch akzeptable weichmachende Hilfsstoffe herabgesetzt. Geeignete polymere Bindemittel sind beispielsweise beschrieben in WO 97/15291.

Als polymere Bindemittel werden für die Schmelzextrusion pharmazeutischer Wirkstoffe bevorzugt eingesetzt: Polymere oder Copolymerisate von N-Vinylpyrrolidon, Eudragittypen (Acrylharze) oder Cellulosen. Dabei sind besonders bevorzugt: Polyvinylpyrrolidon (PVP), Copolymerisate von N-Vinylpyrrolidon und Vinylestern, wie Vinylacetat, Poly(hydroxyalkylacrylate), Poly(hydroxyalkylmethacrylate), Polyacrylate, Polymethacrylate, Alkylcellulosen oder Hydroxyalkylcellulosen.

Die extrudierbare Mischung kann neben dem polymeren Bindemittel und dem (oder den) Wirkstoff(en) auch übliche Zusätze enthalten, beispielsweise Weichmacher, Schmiermittel, Fließmittel, Farbstoffe, Stabilisatoren oder Netz-, Konservierungs-, Spreng- Adsorptions-, Formentrenn- und Treibmittel. Ebenso können übliche galenische Hilfsmittel, z.B. Streckmittel bzw. Füllstoffe enthalten sein. Geeignete Zusätze und galenische Hilfsmittel sind beispielsweise beschrieben in WO 97/15291.

### Beispiele

### Vergleichsbeispiel 1

Eine Mischung, die als Wirkstoff 48 Gew.-% Verapamil-Hydrochlorid und als Hilfsstoffe Hydroxypropylcellulose, Methylhydroxypropylcellulose und Lecithin-Pulver enthielt, wurde über einen Doppelschneckenextruder (ZSK-58; Fa. Werner und Pfleiderer) zu einer homogenen Schmelze verarbeitet. Der Massendurchsatz betrug 120 kg/h. Die Materialtemperatur kurz vor der Austrittsdüse des Extruders betrug ca. 120-130 °C. Die Schmelze wurde über eine Breitschlitzdüse in Form eines Fells ausgetragen und in einem nachgeschalteten Formwalzen-Kalander zu länglichen Tabletten (ohne Bruchkerbe, ca. 20 mm lang, ca. 5 mm dick) geformt. Die Tabletten verließen den Kalander in Form eines zusammenhängenden Tablettenbands. Auf einem Transportband von etwa 4 m Gesamtlänge kühlten die Tablettenbänder durch Wärmeabstrahlung an die Umgebungsluft ab.

50 kg des erhaltenen Tablettenbands wurden am Ende des Transportbands manuell in kleinere Bandstücke zerteilt, die dann in einen Driacoater-Kessel (Fa. Driam) gefüllt wurden. Bei einer Umdrehungszahl von 20 U/min der Trommel erfolgte die Vereinzelung und Entgratung. Der gesamte Vorgang benötigte etwa 40 Minuten. Alle Tabletten konnten vereinzelt und entgratet werden.

### Vergleichsbeispiel 2

Der Versuch erfolgte wie in Vergleichsbeispiel 1 angegeben, aber die Kalandrierung erfolgte zu länglichen Tabletten (identische Länge/Breite) mit einer Bruchkerbe in der Mitte der Tablette. Die Vereinzelung und Entgratung im Driacoater bewirkte, daß etwa 10-30 % der Tabletten bereits während der Rotation im Driacoater zerbrachen.

### Beispiel 1

Der Versuch erfolgte wie in Vergleichsbeispiel 1 angegeben, aber mit folgenden Änderungen:
- Das Transportband enthielt am Ende eine Bürstenwalze (Durchmesser ca. 9 cm), die über einen separaten Antriebsmotor angetrieben wurde. Die Rotationsgeschwindigkeit der Brechwalzen war auf die Fördergeschwindigkeit des Transportbands abgestimmt.
- die Kalandrierung erfolgte zu brechbaren Tabletten, die in der Mitte eine Bruchkerbe besaßen (Bruchkerben-Geometrie wie in Vergleichsbeispiel 2).

Die Tablettenbänder konnten mit Hilfe der Bürstenwalzen gut vereinzelt werden. 50 kg der bereits vereinzelten Tabletten wurden dann in einen Driacoater gegeben und bei einer Trommeldrehzahl von 5-10 U/min entgratet. Die Entgratung war bereits nach 10 Minuten beendet. Ein erhöhter Anteil von zerbrochenen Tabletten konnte nicht festgestellt werden.

### Beispiel 2

Der Versuch erfolgte wie in Beispiel 1 angegeben, allerdings war am Ende des Transportbandes anstelle einer Bürstenwalze eine 450 mm lange Lamellenwalze aus Kunststoff mit 9 in Walzenlängsachse orientierten Lamellen angeordnet (entsprechend der Darstellung der Figur 5). Der Durchmesser des zylindrischen aus POM bestehenden Grundkörpers der Walze betrug 75 mm. Die den Walzenkörper ca. 15 mm tief eingesetzten Lamellen auch Weich-PVC ragten ca. 20 mm über den walzenmantel hinaus.

Die Bänder aus Tabletten mit Bruchkerben konnten mit Hilfe der Lamellenwalzen gut vereinzelt werden. 400 kg der bereits vereinzelten Tabletten wurden dann in einen Driacoater gegeben und bei einer Trommeldrehzahl von 5-10 U/min entgratet. Auch hier wurde kein erhöhter Anteil von zerbrochenen Tabletten festgestellt.

## Patentansprüche

1. Verfahren zum Herstellen von Tabletten mittels Schmelzextrusion, bei dem man eine extrudierbare Mischung erwärmt und in Form eines kontinuierlichen Produktstranges extrudiert, den noch verformbaren Produktstrang zu einem kontinuierlichen Tablettenband verpreßt, wobei die einzelnen Tabletten des Bandes durch Produktstege miteinander verbunden sind, das Tablettenband abkühlen läßt und die Tabletten schließlich vereinzelt und entgratet, **dadurch gekennzeichnet, daß** man zunächst die Tabletten in einem kontinuierlichen Prozeß mechanisch vereinzelt, dann die vereinzelten Tabletten weiter transportiert und sie anschließend entgratet.

2. Verfahren gemäß Anspruch 1, **dadurch gekennzeichnet, daß** man zum Vereinzeln der Tabletten eine Kraft mit einer zur Ebene des Tablettenbandes senkrechten Komponente auf das Tablettenband einwirken läßt.

3. Verfahren gemäß einem der Ansprüche 1 oder 2, **dadurch gekennzeichnet, daß** man zum Vereinzeln der Tabletten eine Kraft mit einer zur Ebene des Tablettenbandes parallelen Komponente auf das Tablettenband einwirken läßt.

4. Verfahren gemäß einem der Ansprüche 2 oder 3, **dadurch gekennzeichnet, daß** man die senkrechte Kraftkomponente durch Auslenken des erstarrten Tablettenbandes aus seiner Transportebene erzeugt.

5. Verfahren gemäß einem der Ansprüche 3 oder 4, **dadurch gekennzeichnet, daß** die parallele Kraftkomponente durch Ausüben einer Zugkraft auf das erstarrte Tablettenband erzeugt.

6. Vorrichtung zum Herstellen von Tabletten, insbesondere zur Durchführung des Verfahrens nach einem der Ansprüche 1 bis 5, mit wenigstens einem Extruder (10), einem dem Extruder nachgeordneten Mittel (20) zum Formen eines Tablettenbandes (14), einem dem Formungsmittel (20) nachgeordneten ersten Transportmittel (30) für das Tablettenband (14) und mit Mitteln (40,50) zum Vereinzeln und Entgraten der Tabletten,**dadurch gekennzeichnet, daß** die Mittel zum Vereinzeln und Entgraten der Tabletten wenigstens ein dem ersten Transportmittel (30) nachgeordnetes, kontinuierlich arbeitendes Vereinzelungsmittel (40) und wenigstens ein dem Vereinzelungsmittel nachgeordnetes und von diesem räumlich getrenntes Entgratungsmittel (50) umfassen.

7. Vorrichtung gemäß Anspruch 6, **dadurch gekennzeichnet, daß** das Vereinzelungsmittel (40) wenigstens eine drehbare Walze (41) zur Auslenkung des Tablettenbandes (14) aus einer Transportebene (34) der ersten Transportmittel (30) umfaßt.

8. Vorrichtung gemäß Anspruch 7, **dadurch gekennzeichnet, daß** das Vereinzelungsmittel (40 )zwei gegeneinander pressbare, gegenläufig rotierende Walzen (41,42) umfaßt.

9. Vorrichtung gemäß einem der Ansprüche 6 bis 8, **dadurch gekennzeichnet, daß** das Vereinzelungsmittel (40) wenigstens eine Bürsten- oder Noppenwalze (41) umfaßt.

10. Vorrichtung gemäß einem der Ansprüche 6 bis 9, **dadurch gekennzeichnet, daß** das erste Transportmittel (30) Mittel (70) zum Kühlen des extrudierten Tablettenbandes umfaßt.

11. Vorrichtung gemäß einem der Ansprüche 6 bis 10, **dadurch gekennzeichnet, daß** zwischen den vereinzelungsmitteln (40) und den Entgratungsmitteln (50) ein zweites Transportmittel (60) vorgesehen ist, das eine Rüttel- oder Vibrationseinrichtung (61) umfaßt.

## Claims

1. A process for producing tablets by melt extrusion, in which an extrudable mixture is heated and extruded in the form of a continuous product strip, the still deformable product strip is compressed to a continuous tablet belt, the individual tablets in the belt being connected together by product webs, the tablet belt is allowed to cool, and the tablets are finally singulated and deflashed, wherein firstly the tablets are mechanically singulated in a continuous process, and then the singulated tablets are transported further and subsequently deflashed.

2. A process as claimed in claim 1, wherein a force with a component perpendicular to the plane of the tablet belt is allowed to act on the tablet belt for singulation of the tablets.

3. A process as claimed in either of claims 1 or 2, wherein a force with a component parallel to the plane of the tablet belt is allowed to act on the tablet belt for singulation of the tablets.

4. A process as claimed in either of claims 2 or 3, wherein the perpendicular force component is generated by diverting the solidified tablet belt out of its transport plane.

5. A process as claimed in either of claims 3 or 4, wherein the parallel force component is generated by exerting a traction force on the solidified tablet belt.

6. An apparatus for producing tablets, in particular for carrying out the process as claimed in any of claims 1 to 5, having at least one extruder (10), having means (20), downstream of the extruder, for shaping a tablet belt (14), having first transport means (30), downstream of the shaping means (20), for the tablet belt (14) and having means (40, 50) for singulating and deflashing the tablets, wherein the means for singulating and deflashing the tablets comprise at least one continuously operating singulating means (40), downstream of the first transport means (30), and at least one deflashing means (50), downstream of the singulating means and spatially separate therefrom.

7. An apparatus as claimed in claim 6, wherein the singulating means (40) comprises at least one rotatable roller (41) for diverting the tablet belt (14) out of a transport plane (34) of the first transport means (30).

8. An apparatus as claimed in claim 7, wherein the singulating means (40) comprises two counter-rotating rollers (41, 42) which can be pressed against one another.

9. An apparatus as claimed in any of claims 6 to 8, wherein the singulating means (40) comprises at least one brush roller or embossed roller (41).

10. An apparatus as claimed in any of claims 6 to 9, wherein the first transport means (30) comprises means (70) for cooling the extruded tablet belt.

11. An apparatus as claimed in any of claims 6 to 10, wherein a second transport means (60) is provided between the singulating means (40) and the deflashing means (50) and comprises a shaking or vibrating unit (61).

## Revendications

1. Procédé pour la préparation de comprimés par extrusion à l'état fondu dans lequel on chauffe un mélange extrudable et on l'extrude sous forme d'un boudin de produit continu, on comprime le boudin de produit encore déformable en un ruban continu de comprimés dans lequel les comprimés individuels sont reliés entre eux par des barrettes de produit, on laisse refroidir le ruban de comprimés et finalement on sépare les comprimés et on les ébarbe, **caractérisé par le fait que** l'on sépare les comprimés dans une opération continue mécanique puis on transporte les comprimés individuels et on les ébarbe.

2. Procédé selon la revendication 1, **caractérisé par le fait que**, pour séparer les comprimés, on exerce sur le ruban de comprimés une force ayant une composante perpendiculaire au plan du ruban de comprimés.

3. Procédé selon l'une des revendications 1 ou 2, **caractérisé par le fait que**, pour séparer les comprimés, on exerce sur le ruban de comprimés une force ayant une composante parallèle au plan du ruban de comprimés.

4. Procédé selon l'une des revendications 2 ou 3, **caractérisé par le fait que** l'on produit la composante perpendiculaire de la force en déviant le ruban de comprimés solidifié de son plan de transport.

5. Procédé selon l'une des revendications 3 ou 4, **caractérisé par le fait que** l'on produit la composante parallèle de la force en exerçant une force de traction sur le ruban de comprimés solidifié.

6. Appareillage pour la fabrication de comprimés, en particulier pour la mise en oeuvre du procédé selon l'une des revendications 1 à 5, qui comprend au moins une extrudeuse (10), un dispositif (20) placé après l'extrudeuse servant à former un ruban de comprimés (14), un premier dispositif de transport (30) disposé après le dispositif de formage (20) pour le ruban de comprimés (14) avec des dispositifs (40, 50) servant à séparer et à ébarber les comprimés, **caractérisé par le fait que** les dispositifs servant à séparer et ébarber les comprimés comprennent au moins un dispositif de séparation opérant en continu (40) placé derrière le premier dispositif de transport (30) et au moins un dispositif d'ébarbage (50) placé derrière le dispositif de séparation et séparé de celui-ci dans l'espace.

7. Appareillage selon la revendication 6, **caractérisé par le fait que** le dispositif de séparation (40) comprend au moins un cylindre rotatif (41) qui sert à dévier le ruban de comprimés (14) d'un plan de transport (34) du premier dispositif de transport (30).

8. Apareillage selon la revendication 7, **caractérisé par le fait que** le dispositif de séparation (40) comprend deux cylindres rotatifs (41, 42) tournant en sens contraire et qui peuvent être pressés l'un contre l'autre.

9. Appareillage selon l'une des revendication 6 à 8, **caractérisé par le fait que** le dispositif de séparation (40) comprend au moins un cylindre à brosses ou à boutons (41).

10. Apareillage selon l'une des revendications 6 à 9, **caractérisé par le fait que** le premier dispositif de transport (30) comprend des dispositifs (70) servant à refroidir le ruban de comprimés extrudé.

11. Appareillage selon l'une des revendications 6 à 10, **caractérisé par le fait que**, entre le dispositif de séparation (40) et le dispositif d'ébarbage (50) on a prévu un deuxième dispositif de transport (60) qui comprend un mécanisme à secousses ou à vibrations (61).
